# EUROPEAN PATENT APPLICATION

(11) **EP 4 183 382 A1**
(43) Date of publication of application: **24.05.2023**
(21) Application number: 21209374.4
(22) Date of filing: 19.11.2021
(51) Int. Cl.: A61F 13/551, A61F 13/475, A61F 13/49, A61F 13/496, A61F 13/532, A61F 13/539

(54) **ARRAY OF DISPOSABLE ABSORBENT ARTICLES**

(71) Applicant: Ontex BV, 9255 Buggenhout (BE); Ontex Group NV, 9320 Erembodegem (BE)
(72) Inventor: COBBAERT, Dries, 1770 Liedekerke (BE); Van Sande, Nathalie, 9688 Maarkedal (BE)
(74) Representative: Larangé, Françoise

(57) **Abstract**

The present invention relates to an array of disposable absorbent articles adapted for different activities, the array comprising a first package comprising a plurality of pant-type absorbent articles (10), configured to be worn about the lower torso of a wearer and a second package comprising a plurality of pad-type absorbent articles (10'), configured to be fastened to an undergarment, wherein the absorbent core (1,1') of said pant-type and said pad-type absorbent articles (10,10') comprises absorbent material selected from the group consisting of cellulose fibers, superabsorbent polymers and combinations thereof, said absorbent material being contained within at least one core wrap substrate enclosing said absorbent material, and wherein a top layer of said core wrap is adhered to a bottom layer of said core wrap to form one or more channels (4) substantially free of said absorbent material.

## Description

### TECHNICAL FIELD

The present disclosure pertains to an array of products comprising two or more disposable absorbent articles of different type. Specifically, the array comprises: a first package comprising a plurality of disposable pant-type absorbent articles and a second package comprising a plurality of disposable incontinence pad-type absorbent articles. More generally, the present disclosure pertains to the technical field of absorbent hygiene products and relates to an absorbent core that can be used within an article for absorbing body fluids and exudates. In particular, the present disclosure relates to absorbent articles (or garments), such as disposable incontinence pants and incontinence pads. More particularly the present disclosure relates to said disposable absorbent articles comprising absorbent cores having one or more channels therethrough.

### BACKGROUND

Disposable absorbent articles, for example in the form of incontinence liners, baby diapers and sanitary napkins, are well known. The general purpose of such absorbent articles is to absorb, distribute and store various types of body exudates, while providing a high level of comfort and sense of dryness to the wearer during use of the absorbent article. Also, such an absorbent article is configured to prevent the wearer from getting the clothes soiled by such body exudates.

Absorbent articles in the form of incontinence articles are used to protect a wearer against urine leakage. An incontinence article can be configured for example as an incontinence pad, a pant diaper, a sanitary pant or an incontinence pant adapted for use by a baby, child or adult, male or female user. Also, an incontinence article is designed with an absorption capacity which is adapted to absorb the discharges that is expected to be released into the article when it is worn. Incontinence articles are used to assist persons with incontinence so that they can maintain a normal lifestyle without any inconvenience caused by incontinence.

More specifically, there are person who use different incontinence products, namely who use both pad-type absorbent articles, also referred herein to as light incontinence products and pant-type absorbent articles products. These persons use light incontinence products in the greater part of their daily activities but do not feel comfortable in using such articles during sport activities because due to all the movements the light incontinence pad absorbent article is prone to shift so for these kind of activities they use pant-type absorbent articles instead. Therefore, it might be opportune for these users to have both light incontinence products, *i.e.* pad-type products, and pant-type products in the same array. It can be particularly beneficial for these users that both the pad-type products and the pant-type products both comprise absorbent articles with channels, more specifically pad-type products and the pant-type products both comprise absorbent articles with absorbent cores comprising channels that are substantially free of absorbent material. Indeed, should only the pant-type product comprise channels the user might start doubting their choice to use both concepts because the absorption speed would be higher for pant-type product when compared to pad-type product. On the other hand, the user doesn't want to use pant-type products exclusively because pad-type products offer more discreteness and are adapted for daily life activities. That's why it is preferable to have pad-type products with channels in the same array as the pant-type products with channels.

Prior art focuses on the difference between sizes namely to compensate anatomical differences between men and women with publications such as EP2144583, EP3600197, EP3544561 all disclosing array of packages comprising one type of products with products of different sizes. In other words, there is no array of products adapted for a user based on the different potential activities that this user might undertake.

The present disclosure aims to provide an array of absorbent articles comprising two or more disposable channeled absorbent articles of different type. Specifically, the array of absorbent articles according to the present disclosure comprises a first package comprising a plurality of first disposable pant-type absorbent articles comprising channels and a second package comprising a plurality of second disposable incontinence pad-type absorbent articles comprising channels so that a user can have products adapted for sport activities (pant-type articles) and discrete articles for daily activities (pad-type articles). Additionally, these absorbent articles utilizing a channeled core particularly designed to provide exceptionally fast absorption and good liquid distribution throughout the entire core, leading to improved absorption capacity and low rewet that provides to the consumer an enhanced perception of dryness. It further offers an improved fit to the wearer, thereby providing comfort and minimizing the risk of leakage.

### SUMMARY

Array of disposable absorbent articles adapted for different activities, the array comprising:
a first package comprising a plurality of pant-type absorbent articles, configured to be worn about the lower torso of a wearer, said pant-type absorbent articles comprising:
   a chassis comprising a front waist region, a back waist region each of said regions being provided with longitudinal edges and a transversal edge; and a crotch region extending between said front and back waist regions; preferably said front waist region and back waist region being arranged or configured to be attached together or to one another;
   a liquid-absorbent member extending across the crotch region in the direction of a longitudinal center line of the absorbent article into the front and back waist regions, the liquid-absorbent member comprising a liquid permeable topsheet arranged on a body-facing side, a liquid impermeable backsheet arranged on a garment-facing side and an absorbent core arranged in between said topsheet and backsheet; and
   wherein the crotch region, the front waist region and the back waist region define leg openings of the absorbent article, the front waist region and the back waist region having an edge contour extending starting from the longitudinal edges towards a transverse center line of the crotch region;
   wherein said front waist region and back waist region comprise first elastification means, and, in an area of the front waist region and the back waist region on the crotch side facing the leg openings, second elastification means are provided, said second elastification means extend starting from longitudinal edges towards the longitudinal center line of the absorbent article; and
a second package comprising a plurality of pad-type absorbent articles, configured to be fastened to an undergarment, said pad-type absorbent article comprising:
   a liquid permeable topsheet arranged on a body-facing side;
   a liquid impermeable backsheet arranged on a garment-facing side provided with fastening means to secure the pad-type absorbent article to an undergarment; and
   an absorbent core arranged in-between said topsheet and backsheet,
wherein the absorbent core of said pant-type and said pad-type absorbent articles comprises absorbent material selected from the group consisting of cellulose fibers, superabsorbent polymers and combinations thereof, said absorbent material being contained within at least one core wrap substrate enclosing said absorbent material, and wherein a top layer of said core wrap is adhered to a bottom layer of said core wrap to form one or more channels substantially free of said absorbent material.

The one or more channels are arranged within the inner periphery of the absorbent core, *i.e.* the inner, or interior, space defined by the periphery of the absorbent core, such that each of the channel(s) is bounded, or surrounded, by absorbent material. Additionally, the length of the channels may be from 10% to 95% of the length of the absorbent core so that again the one or more channels are bounded by absorbent material.

The array of absorbent articles according to the disclosure provides the user the possibility to acquire absorbent articles that are adapted for any kind of activity. By having common features, specifically channel(s), other features can be modified without impacting absorbency.

According to an embodiment, the one or more channels of the absorbent core of the pant-type absorbent article have the same shape as the one or more channels of the absorbent core of the pad-type absorbent article.

According to an embodiment, the pant-type absorbent articles and the pad-type absorbent articles each comprise symmetrical channel(s).

According to an embodiment, the one or more channels of the absorbent core of the pant-type absorbent article have a different shape than the one or more channels of the absorbent core of the pad-type absorbent article.

According to an embodiment, each pant-type absorbent article comprises asymmetrical channel(s) and each pad-type absorbent article comprises symmetrical channel(s).

Pad-type absorbent articles are usually symmetrical in the sense that there is no given front end or rear end and the pad can be placed in any direction within the undergarment. By comprising a symmetrical channel shape, this advantage is kept, as consumers don't need to wonder how to position the product. Whereas for Pant-type absorbent articles there is a clear difference between the front end and back end of the product defined by the cut of the chassis. Pant-type absorbent articles can thus comprise asymmetrical channel(s), for example said asymmetrical channels being gender-specific, the point of insult differing between men and women. Of course, pant-type absorbent articles can also comprise symmetrical channel(s) for uniformity that are adapted for both women and men.

According to an embodiment, the pant-type absorbent articles comprise a first channel area to core area ratio and the pad-type absorbent articles comprise a second channel area to core area ratio, wherein the first channel area to core area ratio is lesser than the second channel area to core area ratio, preferably at least 15% lesser, preferably at least 20% lesser, more preferably at least 30% lesser, more preferably between 40% and 70% lesser.

It is preferable to have optimal balance for the core between channel area and core area, i.e. area free of channel. Enlarging the channel area might ensure a better performance in terms of acquisition speed but it results in having less space for the area free of channel and thus a smaller core area to apply the absorbent material, ultimately leading to lack of performance in terms of absorbency. In contrast, if the channel area is too small, it complicates the process to get well defined channels and thus good channel bonding and the acquisition speed would be reduced. It is preferable that the pant-type absorbent articles present better absorbency performance in comparison to pad-type absorbent articles depending on the activity considered, therefore in the array of product the pant-type absorbent articles should present a smaller channel area to core area ratio than the pad-type absorbent articles. Indeed, when doing outdoor sport activities it can be complicated to have access to restrooms to dispose of and replace absorbent articles, it is preferable for the pant-type absorbent articles to have better absorbency properties than the pad-type absorbent articles that can be changed more easily.

According to an embodiment, said absorbent core of said pant-type and/or said pad-type absorbent article having a first and second longitudinal edge, a front and back transverse edge, and a longitudinal center line dividing the absorbent core in a first longitudinal portion and a second longitudinal portion on either side of the longitudinal center line, said absorbent core having a transverse center line dividing the absorbent core in a front portion and a back portion on either side of the transverse center line, thereby forming four quadrants, wherein at least one channel follows a substantially continuous path from any point of said channel to any other point of the same channel, **characterized in that** said channel comprises at least:
- one longitudinally extending central section coincident with the longitudinal center line,
- one longitudinally extending lateral section in each of the four quadrants,
- four diagonally extending diagonal sections, diverging from the central section end points towards the lateral sections in each quadrant and connecting said central and lateral sections, and
- one transversally extending closing section, in one of the front or back portions, joining each of the two lateral sections end points closest to the absorbent core respective front or back transverse edge.

According to an embodiment, the absorbent core of said pant-type and/or said pad-type absorbent article comprises a channel comprising a second transversally extending closing section, in the other of the front or back portions, joining each of the two lateral sections end points closest to the absorbent core respective front or back transverse edge.

According to an embodiment, said channel consists of one central section, four lateral sections, four diagonal sections and one or two closing section(s).

According to an embodiment, the central section is longitudinally centered on a point which is at a distance from the front transverse edge along the longitudinal center line of between 30% and 55% of the absorbent core length.

According to an embodiment, the central section has a length of between 4 and 25% of the absorbent core length, wherein the ratio between the central section length to the absorbent core length is greater for the absorbent core of the pant-type absorbent article than for the absorbent core of the pad-type absorbent article.

According to an embodiment, the length of the channel taken along the longitudinal center line is between 50 and 90% of the absorbent core length wherein the ratio between the channel length taken along the longitudinal center line to the absorbent core length for the absorbent core of the pant-type absorbent article is substantially equal to said ratio for the absorbent core of the pad-type absorbent article.

According to an embodiment, the length of the channel taken along the longitudinal center line is between 70 and 95% of the absorbent core length.

According to an embodiment, the absorbent core is free of any channel or channel section extending up to the front and back transverse edges of the absorbent core.

According to an embodiment, the lateral sections of the first longitudinal portion are aligned on a first longitudinal axis parallel to the longitudinal center line, and the lateral sections of the second longitudinal portion are aligned on a second longitudinal axis parallel to the longitudinal center line.

According to an embodiment, the liquid permeable topsheet is treated with a hydrophilic agent, preferably comprising anionic and/or zwitterionic agents.

According to an embodiment, only part of the topsheet is treated and the treated zone is positioned over the channel, preferably the width of the treated zone corresponding to the maximum width of the channel, as measured along a direction parallel to the transverse center line.

Each type of absorbent articles comprised in the array of disposable absorbent articles according to the invention, comprise a liquid permeable topsheet, a liquid impermeable backsheet and an absorbent core positioned in between said topsheet and said backsheet. The absorbent core has a first and second longitudinal edge and a front and back transverse edge. It has a longitudinal center line dividing the absorbent core in a first longitudinal portion and a second longitudinal portion on either side of the longitudinal center line. It also has a transverse center line dividing the absorbent core in a front portion and a back portion on either side of the transverse center line. The longitudinal center line and the transverse center line together create four quadrants within the absorbent core. The absorbent core comprises absorbent material selected from the group consisting of cellulose fibers, superabsorbent polymers and combinations thereof, and the absorbent material is contained within at least one core wrap substrate enclosing said absorbent material. The top layer of said core wrap is adhered to a bottom layer of said core wrap to form one or more channels substantially free of said absorbent material. At least one channel follows a substantially continuous path from any point of said channel to any other point of the same channel. Said channel comprises at least:
- one longitudinally extending central section coincident with the longitudinal center line,
- one longitudinally extending lateral section in each of the four quadrants,
- four diagonally extending diagonal sections, diverging from the central section end points towards the lateral sections in each quadrant and connecting said central and lateral sections, and
- one transversally extending closing section, in one of the front or back portions, joining each of the two lateral sections end points closest to the absorbent core respective front or back transverse edge.

We have found that having a longitudinally extending central section coincident with the longitudinal center line, i.e. of a certain length rather than a crossing point, greatly improves the absorption time, providing shorter absorption times under pressure for example. We also believe that this longitudinally extending central section is advantageous for providing some tolerance in the placement of the absorbent article, as the length of the section may cover various positioning of the miction point.

The lateral sections greatly participate in the distribution of the fluid in the fourth quadrants of the core, for a distribution through the entire core. Their extension in the longitudinal direction helps guiding the fluid to the front and back of the absorbent core.

We have found that the diagonal sections help in providing a good cup shape and 3D bending of the absorbent article once worn. This provides comfort to the user and may also reduce the risk of leakage through the longitudinal edges in the crotch region. We have indeed noted that with channels of the prior art which mainly extend longitudinally as a pair of parallel channels, the absorbent article may tend to wind on itself in the crotch region, i.e. the longitudinal edges wrapping over the longitudinal center line between the legs of the user, thereby increasing the risk of leakage on the sides of the absorbent article between the user's legs. The diagonal sections may help in preventing such wrapping.

Finally, we have found that having a transversally extending closing section may help the distribution of liquid beyond simply the end points of the lateral sections closest to the transverse edges. The closing section may allow distribution of fluid beyond the closing section itself, closer to the transverse edge of the core.

A continuous channel made of at least these essential channel sections provides fast absorption, good liquid distribution throughout the entire core, improved fit and comfort and minimizes the risk of leakage. It also contributes to limit over-saturation of the core in the portion of fluid discharge. Without wishing to be bound by theory it is believed that the fact that the fluid is distributed across the core and immediately away from the fluid discharge position, a perception of dryness and skin comfort is provided to the wearer, as well as an impression of longer lasting dryness by the user.

Other objects and advantages of this invention will become apparent hereinafter.

### BRIEF DESCRIPTION OF THE FIGURES

Fig. 1 illustrates a diagrammatic top view of a pant-type absorbent article comprised in the first package included in the array of disposable absorbent articles.
Fig. 2 illustrate a diagrammatic top view of a pad-type absorbent article comprised in the second package included in the array of disposable absorbent articles.
Fig. 3 to 6 show diagrammatic top views of absorbent cores according to embodiments herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious topsheet, a backsheet joined to the topsheet, and an absorbent core positioned and held between the topsheet and the backsheet. The topsheet is operatively permeable to the liquids that are intended to be held or stored by the absorbent article, and the backsheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

An absorbent article, such as an incontinence pant, comprises a front waistband region, a back waistband region, an intermediate crotch region which interconnects the front and rear waistband regions. When used herein, reference to a "front" portion refers to that part of the absorbent article which is generally located on the front of a subject, such as an infant or adult, when in use. Reference to the "rear" or "back" "portion" or "section" refers to the portion of the absorbent article generally located at the rear of the subject, such as an infant or adult, when in use, and reference to the "crotch" portion refers to that portion which is generally located between the legs of subject, such as an infant or adult, when in use. The crotch region is an area where repeated fluid surge typically occurs, within the absorbent article assembly.

"Front", "rear" or "back", and "crotch" portions of the absorbent core as used herein typically refer to portions of the absorbent core that are proximal to respective portions of the absorbent article. For example, the "front" portion of the core is that which is most proximal to the front of the subject when worn, the "rear or back" portion of the core is that which is most proximal to the rear or back of the subject when worn, and the "crotch" portion of the core is the middle portion of the absorbent core between the "front" and "rear or back" portions.

Preferably, an incontinence pant or an incontinence pad comprises a liquid permeable "topsheet", a liquid impermeable "backsheet", and an "absorbent medium" disposed between the topsheet and the backsheet. The topsheet, backsheet and the absorbent medium could be made from any suitable material known to the person skilled in the art. The topsheet is generally located at or near the bodyside surface of the article, while the backsheet is generally located at or near the garment-side surface of the article. Optionally, the article may comprise one or more separate layers which are in addition to the backsheet and are interposed between the backsheet and the absorbent medium. Topsheet and backsheet are connected or otherwise associated together in an operable manner.

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means as are known in the art.

"Acquisition and distribution layer", "ADL", "Acquisition and distribution system" or "surge management portion" refers to a sub-layer which preferably is a nonwoven wicking layer under the topsheet of an absorbent product (or absorbent article), which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the topsheet and the retention portion.

The term "adhesive" as used herein is intended to refer to any suitable hot melt, water or solvent borne adhesive that can be applied to a surface of a product component of the present disclosure. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, an "airformed web" refers to a material comprising cellulosic fibers such as those from fluff pulp that have been separated, such as by a hammermilling process, and then deposited on a porous surface without a substantial quantity of binder fibers present. Airfelt materials used as the absorbent core in many incontinence pants or pads, for example, are a typical example of an airformed material.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present disclosure can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present disclosure. Binder can be added to an airformed web in liquid form (e. g., an aqueous solution or a melt) by spray nozzles, direction injection or impregnation, vacuum drawing, foam impregnation, and so forth. Solid binder particles can also be added by mechanical or pneumatic means.

As used herein, an "air-through-bonded" nonwoven, is a nonwoven structure primarily formed by a process that comprises the application of heated air to the surface of the nonwoven fabric. During the through air bonding process, heated air flows through holes in a plenum above the nonwoven material. Unlike hot ovens, which push air through the material, the through air process uses negative pressure of suction to pull the air through an open conveyor apron holding nonwoven as it is drawn through the oven. Pulling air through the material allows the rapid and even transmission of heat to minimize distortion of the nonwoven material. The binding agents used in the through air bonding process include crystalline binder fibers and powders, which melt to form molten droplets throughout the cross-section of the nonwoven. As the material is cooled, bonding occurs at these droplet points.

As used therein, the term "associated" encompasses configurations in which topsheet is directly joined to backsheet by affixing topsheet directly to backsheet, and configurations wherein topsheet is joined to backsheet by affixing topsheet to intermediate members which in turn are affixed to backsheet. Topsheet and backsheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix topsheet to backsheet. It should be readily appreciated that the above-described attachment means may also be employed to interconnect and assemble together the various other component parts of the article described herein.

The term "backsheet" or "back sheet" refers to a material forming the outer cover of the absorbent article. The backsheet prevents the exudates contained in the absorbent structure from wetting articles such as bedsheets and overgarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapor to escape from the absorbent material, while still preventing liquids from passing there through. Examples of breathable backsheet materials are porous polymeric films, nonwoven laminates of spunbond and meltblown layers and laminates of porous polymeric films and nonwoven materials.

The term "blend" means a mixture of two or more polymers while the term "alloy" means a sub-class of blends wherein the components are immiscible but have been compatibilized.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to films having a water vapor transmission rate (WVTR) of at least 300 grams/m² - 24 hours.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and

hydroentanglement. Carded thermobonded nonwoven thus refers to a carded nonwoven wherein the bonding is achieved by use of heat.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Coform" as used herein is intended to describe a blend of meltblown fibers and cellulose fibers that is formed by air forming a meltblown polymer material while simultaneously blowing air-suspended cellulose fibers into the stream of meltblown fibers. The coform material may also include other materials, such as superabsorbent particles. The meltblown fibers containing wood fibers are collected on a forming surface, such as provided by a foraminous belt. The forming surface may include a gas-pervious material, such as spunbonded fabric material, that has been placed onto the forming surface.

"Compression" refers to the process or result of pressing by applying force on an object, thereby increasing the density of the object.

"Conventional hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "density" or "concentration" when referring to the absorbent material, in particular the SAP, of a layer, refers to the amount of the absorbent material divided by the surface area of the layer over which the absorbent material is spread out.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" refers to a material, layer, or substrate that is naturally non-elastic, but which has been rendered elastic by, for example, suitably joining an elastic material, layer, or substrate thereto.

The term "elastification means" which is equivalent to "elastic means" refers to a material or element imparting an elasticity or elasticizing a material, layer, or substrate.

"Elongation" means the ratio of the extension of a material to the length of the material prior to the extension (expressed in percent), as represented by the following: "Extension" means the change in length of a material due to stretching (expressed in units of length).

As used herein the term "extensible" means that can be elongated in at least one direction, but not necessarily recoverable.

The term "fabrics" is used to refer to all of the woven, knitted and nonwoven fibrous webs.

As used herein, the term "garment" means any type of apparel which may be worn. This includes diapers, training pants, incontinence products, surgical gowns, industrial workwear and coveralls, undergarments, pants, shirts, jackets and the like.

Many of the known superabsorbent polymer particles exhibit gel blocking. "Gel blocking" occurs when superabsorbent polymer particles are wetted and the particles swell so as to inhibit fluid transmission to other regions of the absorbent structure. Wetting of these other regions of the absorbent member therefore takes place via a very slow diffusion process. In practical terms, this means acquisition of fluids by the absorbent structure is much slower than the rate at which fluids are discharged, especially in gush situations. Leakage from the absorbent article can take place well before the particles of SAP in the absorbent member are even close to being fully saturated or before the fluid can diffuse or wick past the "blocking" particles into the rest of the absorbent member. Gel blocking can be a particularly acute problem if the superabsorbent polymer particles do not have adequate gel strength and deform or spread under stress once the particles swell with absorbed fluid.

The term "graphic" includes, but is not limited to, any type of design, image, mark, figure, codes, words, patterns, or the like. For a product such as a training pant, graphics will generally include objects associated with little boys and little girls, such as multi-colour trucks, airplanes, balls, dolls, bows, or the like.

"Hydroentanglement process" refers to the manufacturing of nonwoven webs. The process involves directing a series of water jets towards a fibrous web which is supported on a moving porous belt. The water jets pass downwards through the mass of fibers and on making contact with the surface of the belt, the jets rebound, and break up: the energy released causes entanglement of the mass of fibers.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

The crotch portion of the absorbent article such as incontinence pants or pads preferably comprises opposite longitudinal side portions which comprise a pair of elasticized, longitudinally-extending "leg cuffs". The leg cuffs are generally adapted to fit about the legs of a wearer when in use and serve as a mechanical barrier to the lateral flow of body exudates. Leg cuffs are elasticized by leg elastics. The incontinence pant further can comprise a front waist elastic and a rear waist elastic. Materials suitable for use in forming leg elastics are known to those skilled in the art. Exemplary of such materials are strands or ribbons of a polymeric, elastomeric material which are adhered to the incontinence pant at the leg cuff while in a stretched position, or which are attached to the incontinence pant while the incontinence pant is pleated, such that elastic constrictive forces are imparted to the leg cuff. Examples of suitable elastomer materials that can be used include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

"Liquid" means a nongaseous substance and/or material that flows and can assume the interior shape of a container into which it is poured or placed.

"Longitudinal" is a direction running parallel to the maximum linear dimension of the article.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.

The term "nonelastic" refers to any material which does not fall within the definition of "elastic" above

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

"Pant body" or "pant" refers to a garment that has a waist opening and a pair of leg openings, similar to shorts, swim wear, or the like. The described garment may or may not have a manually tearable side seam.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp fluff' or "fluff pulp" refers to a material made up of cellulose fibers. The fibers can be either natural or synthetic, or a combination thereof. The material is typically lightweight and has absorbent properties.

The "retention portion" or "liquid absorption layer" is part of the absorbent medium. This portion may comprise a matrix of hydrophilic fibers, such as a web of cellulosic fluff, mixed with particles of high absorbency material. In particular arrangements, the retention portion may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed. For example, the concentrations of superabsorbent particles may be arranged in a non-step-wise gradient through a substantial portion of the thickness of the absorbent structure, with lower concentrations toward the bodyside of the absorbent structure and relatively higher concentrations toward the outerside of the absorbent structure. The superabsorbent particles may also be arranged in a generally discrete layer within the matrix of hydrophilic fibers. In addition, two or more different types of superabsorbent may be selectively positioned at different locations within or along the fiber matrix.

As used herein the term "sheet" or "sheet material" refers to woven materials, nonwoven webs, polymeric films, polymeric scrim-like materials, and polymeric foam sheeting.

The term "spunbond fibers (or layer(s) or nonwovens)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

By "stretch", it is meant that the material has the ability to extend beyond its original size in at least one dimension when subjected to a tensile force (*i.e.,* tension) applied in the direction of that dimension, without breaking the material. An extension of for example 50% means that the material with an initial length of 100mm has reached a length of 150mm. Stretch may be unidirectional, bi-directional, or multi-directional. The specific stretch properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.

As used herein "channels" are fluid distribution means within the absorbent core adapted to favor exudate flow there along and are typically intended to exclude embossing patterns or ducts formed by compression from the meaning thereof and rather include structures that are substantially free of absorbent material instead of comprising compacted absorbent material. Channels herein are formed by joining upper and lower layers of a core wrap as will be described in more detail hereinbelow. Channels preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width generally greater than 1mm, preferably from 5mm to 50mm, more preferably from 6mm to 40mm, more preferably from 8mm to 30mm, even more preferably from greater than 8mm to less than 25mm.

By "substantially", it is meant at least the majority of the structure referred to. For example, with reference to a channel following "a substantially continuous path from any point of said channel to any other point of the same channel", this means that the majority of the channel is interconnected and generally wherein a direct and continuous path can be traced by starting from one point of the channel towards another point of the channel.

Use of the term "substrate" includes, but is not limited to, woven or nonwoven webs, porous films, ink permeable films, paper, composite structures, or the like.

The terms "Superabsorbent" or "high absorbency" refer to materials that are capable of absorbing at least 10 times their own weight in liquid. Superabsorbent materials suitable for use in the present disclosure are known to those skilled in the art, and may be in any operative form, such as particulate form, fibers and mixtures thereof. Generally stated, the "superabsorbent material" can be a water-swellable, organic or inorganic, generally water-insoluble, hydrogel-forming polymeric absorbent material, which is capable of absorbing at least about 15, suitably about 30, and possibly about 60 times or more its weight in physiological saline (e.g. saline with 0.9 wt % NaCI). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal and ammonium salts of polyacrylic acid and polymethacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, maleic anhydride copolymers with vinyl ethers and alpha-olefins, poly(vinyl pyrrolidone), poly(vinylmorpholinone), poly(vinyl alcohol), and the like, and mixtures and copolymers thereof. Other suitable hydrogel-forming polymers include hydrolyzed acrylonitrile grafted starch, acrylic acid grafted starch, and isobutylene maleic anhydride copolymers and mixtures thereof, methyl cellulose, carboxymethyl cellulose, hydroxypropyl cellulose, and the natural gums, such as alginates, xanthan gum, locust bean gum and the like. The hydrogel-forming polymers may be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, irradiation, crystalline domains, covalent bonds, ionic complexes and associations, hydrophilic associations, such as hydrogen bonding, and hydrophobic associations or Van der Waals forces. Mixtures of natural and wholly or partially synthetic absorbent polymers can also be used. Synthetic hydrogel-forming materials typically are xerogels which form hydrogels when wetted. The term "hydrogel", however, has commonly been used to also refer to both the wetted and unwetted forms of the material. The superabsorbent material may be in any of a wide variety of geometric forms. As a general rule, it is preferred that the superabsorbent material be in the form of discrete particles. However, the superabsorbent material may also be in the form of fibers, flakes, rods, spheres, needles, spiral or semi-spiral, cubic, rod-like, polyhedral, or the like. Conglomerates of particles of superabsorbent material may also be used. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibers. Alternatively, the absorbent core may comprise a laminate of fibrous webs and high-absorbency material or other suitable means of maintaining a high-absorbency material in a localized area.

"Superabsorbent polymer particles" or "SAPs" refer to water-swellable, water-insoluble organic or inorganic materials capable, under the most favourable conditions, of absorbing at least about 10 times their weight, or at least about 15 times their weight, or at least about 25 times their weight in an aqueous solution containing 0.9 weight percent sodium chloride. In absorbent articles, such as diapers, incontinent pants or pads, etc., the particle size is typically ranging between 100 to 800 µm, preferably between 300 to 600 µm, more preferably between 400 to 500 µm.

"Tension" includes a uniaxial force tending to cause the extension of a body or the balancing force within that body resisting the extension.

As used herein, the term "thermoplastic" is meant to describe a material that softens when exposed to heat and which substantially returns to its original condition when cooled to room temperature.

The term "topsheet" or "top sheet" refers to a liquid permeable material sheet forming the inner cover of the absorbent article and which in use is placed in direct contact with the skin of the wearer. The topsheet is typically employed to help isolate the wearer's skin from liquids held in the absorbent structure. The topsheet can comprise a nonwoven material, e.g. spunbond, meltblown, carded, hydroentangled, wetlaid etc. Suitable nonwoven materials can be composed of man-made fibers, such as polyester, polyethylene, polypropylene, viscose, rayon etc. or natural fibers, such as wood pulp or cotton fibers, or from a mixture of natural and man-made fibers. The topsheet material may further be composed of two fibers, which may be bonded to each other in a bonding pattern. Further examples of topsheet materials are porous foams, apertured plastic films, laminates of nonwoven materials and apertured plastic films etc. The materials suited as topsheet materials should be soft and non-irritating to the skin and be readily penetrated by body fluid, e.g. urine or menstrual fluid. The inner coversheet may further be different in different parts of the absorbent article. The topsheet fabrics may be composed of a substantially hydrophobic material, and the hydrophobic material may optionally be treated with a surfactant or otherwise processed to impart a desired level of wettability and hydrophilicity.

"Training pants" are available for use by children in the potty-training stage, and are popular with mothers and caretakers. A training pant typically comprises a topsheet, a backsheet, an absorbent medium between the topsheet and the backsheet, and side seams that bond portions of the side edges of the pant together to form waist and leg openings.

As used herein, the term "transverse" or "lateral" refers to a line, axis, or direction which lies within the plane of the absorbent article and is generally perpendicular to the longitudinal direction.

"Ultrasonic welding or bonding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

"Dry-state" refers to the condition in which an absorbent article has not yet been saturated with exudates and/or liquid.

"Wet-state" refers to the condition in which an absorbent article has been saturated with exudates and/or liquid. Typically wherein at least 30ml, preferably at least 40ml, even more preferably at least 50ml, most preferably from 60ml to 800ml, of exudate and/or liquid are contained in the absorbent article.

As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighboring particles.

By the term "concentration" of (e.g. super absorbent polymer) as used herein, is meant the amount of the referred material divided by the surface area of the layer referred to (typically said area being in the plane of the length and width of the core) over or within which the referred material is contained. This may be determined by standard weighing and dimensional measuring methods known in the art and can be expressed in g/mm².

By the term "substantially U-shaped" as used herein, is meant any shape that visually approximates the shape of a "U", such as a "V-shape", a semi-circle, and the like.

By the term "substantially matches the shape of the channel(s)" as used herein, is meant that the feature referred to has an overlapping shape that is visually the same as the channel(s).

By the term "superabsorbent polymer fibers" as used herein, is meant fibers made from superabsorbent polymers (as opposed to particles made therefrom). Examples of suitable fibers for use herein are selected from those of Example 1, Example 2, Example 3, and/or Example 4 (page 5, lines 1-46) of EP3190216A1, incorporated herein by reference. Said fibers typically being used to form nonwoven webs or substrates according to the same referenced application.

By the terms "longitudinally-, transversally-, diagonally- -extending" as used herein, is meant a general orientation substantially parallel respectively to the longitudinal axis, to the transversal axis, and to any axis between them. This covers deviations from the axis used as reference of between 0° and 20°. This may cover straight lines but also curved lines. In the latter case, it is the main general orientation of the curve which is meant to be described.

By the term "center line" or "centre line" as used herein, is meant an axis dividing the element to which it refers, in two portions of equal length on either side of this axis.

The term "symmetrical" refers to an element having, involving, or exhibiting symmetry, *i.e.* correspondence in size, shape, and relative position of parts on opposite sides of a dividing line or median plane or about a center or axis. We can also say said element being made up of exactly similar parts facing each other or around an axis. Naturally, asymmetrical refers to the opposite, meaning an element having parts that fail to correspond to one another in shape, size, or arrangement; *i.e.* lacking symmetry.

Embodiments of the articles and processes according to the disclosure will now be described. It is understood that technical features described in one or more embodiments maybe combined with one or more other embodiments without departing from the intention of the disclosure and without generalization therefrom.

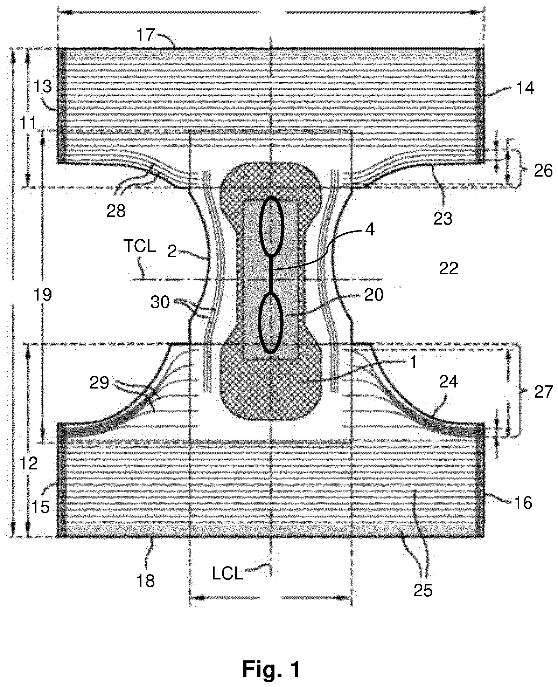

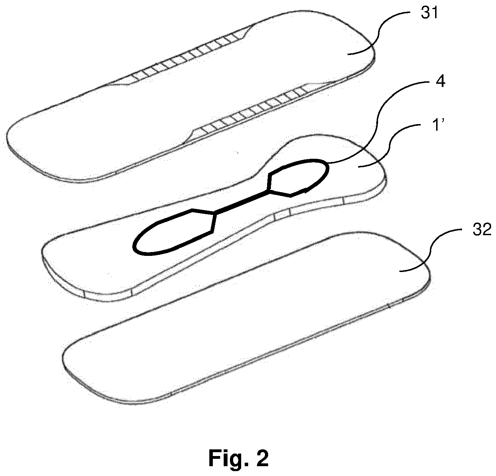

The present invention provides an array of absorbent articles as illustrated in Fig. 1 and 2. The first package comprises a plurality of pant-type absorbent articles 10, illustrated in Fig. 1, suitable to be worn about the lower torso of a wearer and comprising a chassis 2 comprising a front waist region 11 and a back waist region 12, each of said section being provided with longitudinal edges 13, 14, 15, 16 and a transversal edge 17, 18. The pant-type absorbent article 10 further comprises a crotch region 19 extending between the front waist region 11 and the back waist region 12 and is attached, preferably permanently, to the front waist region 11 and the back waist region 12. The pant-type absorbent article 10 comprises a liquid-absorbent member 20 extending across the crotch region 19 in the direction of a longitudinal center line LCL of the pant-type absorbent article 10 into the front and back waist regions 11,12. The liquid-absorbent member 20 comprises a liquid permeable topsheet arranged on a body-facing side, a liquid impermeable backsheet arranged on a garment-facing side and an absorbent core 1 arranged in between said topsheet and backsheet.

The crotch region 19, the front waist region 11 and the back waist region 12 define leg openings 22 of the pant-type absorbent article 10. The front waist region 11 and the back waist region 12 have an edge contour 23,24 which extends starting from the longitudinal edges 13, 14, 15, 16 towards a transverse center line TCL of the absorbent core 1. The front waist region 11 and the back waist region 12 comprise first elastification means 25, and, in an area 26,27 of the front waist region 11 and the back waist region 12 on the crotch side facing the leg openings 22, second elastification means 28,29 are provided. Said second elastification means 28,29 extend starting from the longitudinal edges 13, 14, 15, 16 towards the longitudinal center line LCL of the pant-type absorbent article 10. Preferably, the second elastification means 28,29 extend in a curved shape and are fanning out with increasing separation from each other in the back waist region 12 and with equal separation from each other and parallel to each other in the front waist region 11. In an embodiment, the second elastification means 28 of the front waist region 11 extend starting from the longitudinal edges 13, 14 of said front waist region 11 towards the longitudinal center line LCL of the pant-type absorbent article 10 in a curved shape.

Said second elastification means 28 extends essentially perpendicular to the longitudinal edges 13, 14 of the front waist region 11 and parallel to each other. The second elastification means 28 may continue extension in a curved manner and also parallel to each other towards the longitudinal center line LCL of the absorbent article 10. Preferably, said second elastification means 28 continues extension in the crotch region 19 wherein they are preferably extending essentially perpendicular to the longitudinal center line LCL of the pant-type absorbent article 10. Similarly, said second elastification means 29 extends essentially perpendicular to the longitudinal edges 15, 16 of the back waist region 12 and parallel to each other. The second elastification means 29 may continue extension in a curved manner and also parallel to each other towards the longitudinal center line LCL of the absorbent article 10. Preferably, said second elastification means 29 continues extension in the crotch region 19 wherein they are preferably extending essentially perpendicular to the longitudinal center line LCL of the pant-type absorbent article 10. The second elastification means 28, 29 extend in a curved shape and are fanning out with increasing separation from each other in the back waist region 12 and with substantially equal separation from each other and parallel to each other in the front waist region 11. The second elastification means 29 of the back waist region 12 have a minimum separation of from 1 to 10 mm from each other at the longitudinal edge 15, 16 of said region and a maximum separation of 7 to 35 mm on the crotch region 19 overlapping with the back waist region 12 of the absorbent article. By separation, it is meant the separation of directly neighbouring elastification means.

The front waist region 11 and/or the back waist region 12 can further comprise an elasticized material provided on 1% to 65%, preferably 20% to 60%, more preferably on 40 to 55%, most preferably on at least 50% of the surface area of said front waist region 11 and/or the back waist region 12.

The pant-type absorbent article 10 further comprises leg elastification means 30 in the crotch region 19. Said leg elastification means 30 extend outside and at a given separation from the absorbent core 1 along the leg opening 19.

The pad-type absorbent article 10' illustrated in Fig. 2 is configured to be fastened to an undergarment, said pad-type absorbent article 10' comprises a liquid permeable topsheet 31 arranged on a body-facing side, a liquid impermeable backsheet 32 arranged on a garment-facing side provided with fastening means to secure the pad-type absorbent article 10' to an undergarment and an absorbent core 1' arranged in-between said topsheet 31 and backsheet 32. According to an embodiment, the backsheet 32 comprises on the garment facing side an adhesive material, i.e. a fastening mean, so that the pad-type absorbent article 10' can be secured to an undergarment. The fastening means comprise temporary or reversible fixation means such as, and not limited to, adhesive, hooks or Velcro.

As illustrated in Fig. 1 and 2, the pant-type absorbent article 10 and the pad-type absorbent article 10' of the array of disposable absorbent articles each comprise at least one channel 4, more specifically, the absorbent core 1,1' comprises one or more channels 4 enabling a similar absorbency thus, the user can use either product depending on the activity considered.

The channel(s) 4 of the absorbent core 1 of the pant-type absorbent article 10 has the same shape as the channel(s) 4 of the absorbent core 1' of the pad-type absorbent article 10'. Alternatively, the channel(s) 4 of the absorbent core 1 of the pant-type absorbent article 10 has a different shape than the channel(s) 4 of the absorbent core 1' of the pad-type absorbent article 10'.

The pant-type absorbent articles 10 comprise asymmetrical channel(s) 4 whereas the pad-type absorbent articles 10' comprise symmetrical channel(s) 4.

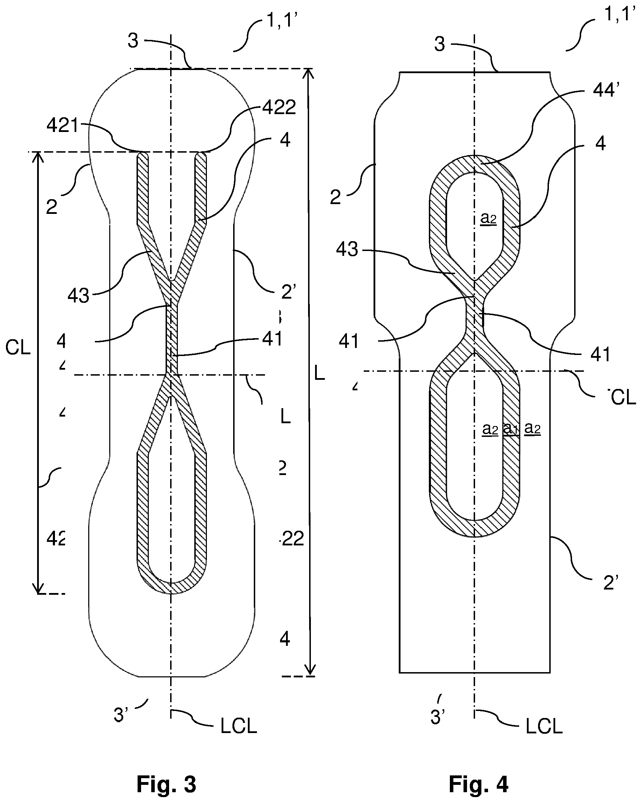

The pant-type absorbent articles 10 comprise a first channel area to core area ratio and the pad-type absorbent articles 10' comprise a second channel area to core area ratio. The first channel area to core area ratio is lesser than the second channel area to core area ratio, preferably at least 15% lesser, preferably at least 20% lesser r, more preferably at least 30% lesser, more preferably between 40% and 70% lesser. The channel area a₁ (see FIG. 4) is the area defined by the channel(s) in the absorbent core 1,1' and the core area a₂ (see FIG. 4) is the area defined by the portion of the absorbent core that is free of channel(s). In other terms, the channel area a₁ is the area of the absorbent core 1,1' that is substantially free of absorbent material whereas the core area a₂ is the area of the absorbent core 1,1' comprising absorbent material. The first channel area to core area ratio (a₁/a₂) is preferably comprised between about 5 % to about 15 %, i.e. the first ratio value is comprised between about 0.05 to about 0.15, whereas the second channel area to core area ratio (a₁/a₂) is preferably comprised between about 10 % to about 20 % (0.1 to 0.2). For example in the array of products, the pant-type absorbent articles 10 comprise a first ratio a₁/a₂ of 11 % whereas the pant-type absorbent articles 10 comprise a second ratio a₁/a₂ of 17%, the first channel area to core area ratio is about 65% lesser than the second channel area to core area ratio.

Each type of absorbent articles comprised in the array of disposable absorbent articles according to the invention, comprises a liquid permeable topsheet, a liquid impermeable backsheet and an absorbent core 1,1' positioned in between said topsheet and said backsheet. The absorbent core 1,1', as for example illustrated in Fig. 3 or Fig. 4, has a first and second longitudinal edge 2,2' and a front and back transverse edge 3,3'. It has a longitudinal center line LCL dividing the absorbent core 1,1' in a first longitudinal portion and a second longitudinal portion on either side of the longitudinal center line LCL. It also has a transverse center line TCL dividing the absorbent core 1,1' in a front portion and a back portion on either side of the transverse center line TCL. The longitudinal center line LCL and the transverse center line TCL together create four quadrants within the absorbent core 1,1'. The absorbent core 1,1' comprises absorbent material selected from the group consisting of cellulose fibers, superabsorbent polymers and combinations thereof, and the absorbent material is contained within at least one core wrap substrate enclosing said absorbent material. The top layer of said core wrap is adhered to a bottom layer of said core wrap to form one or more channels 4 substantially free of said absorbent material. At least one channel 4 follows a substantially continuous path from any point of said channel to any other point of the same channel. Said channel 4 comprises at least:
- one longitudinally extending central section 41 coincident with the longitudinal center line LCL,
- one longitudinally extending lateral section 42 in each of the four quadrants,
- four diagonally extending diagonal sections 43, diverging from the central section end points 411,412 towards the lateral sections in each quadrant and connecting said central 41 and lateral sections 42, and
- one transversally extending closing section 44,44' in one of the front or back portions, joining each of the two lateral sections end points 421,422 closest to the absorbent core respective front or back transverse edge 3,3'.

In an embodiment, as for example illustrated in Fig. 4, the channel 4 comprises a second transversally extending closing section 44,44', in the other of the front or back portions, joining each of the two lateral sections end points 421,422 closest to the absorbent core respective front or back transverse edge 3,3'. This may allow distribution of fluid beyond the second closing section itself, closer to the transverse edge of the core.

In an embodiment, as illustrated in Fig. 3, the channel 4 consists of one central section 41, four lateral sections 42, four diagonal sections 43 and one closing section 44. In that case, preferably, the closing section is in the back portion of the absorbent core, since this portion is generally the most difficult to distribute liquid as being the farthest from the miction point.

In another embodiment, as illustrated in Fig. 4, the channel 4 consists of one central section 41, four lateral sections 42, four diagonal sections 43 and two closing sections 44,44', in order to benefit from the advantages of the closing sections hereinabove described, in both the front and the back of the absorbent core.

As discussed herein, each pant-type absorbent article 10 comprises one or more asymmetrical channel(s) 4 whereas each pad-type absorbent article 10' comprises one or more symmetrical channel(s) 4. The symmetry can be in relation to the transverse center line TCL and/or the longitudinal center line LCL. For the pad-type absorbent article 10', the channel(s) 4 is symmetrical in relation the transverse center line TCL so that the front end and the rear end of the absorbent core will be identical to one other. Additionally, the channel(s) 4 for the pad-type absorbent article 10' can be symmetrical in relation to both the transverse center line and the longitudinal center line LCL to further give the impression of identical rear end and front end. Having channel(s) that are symmetrical in relation to the longitudinal center line LCL alone or not symmetrical at all will give the impression that the front-end is different than the rear end. As for the pant-type absorbent article 10, the front end and rear end is already defined by the shape of the chassis therefore the absorbent core may comprise asymmetrical channel(s). Of course, the absorbent core for the pant-type absorbent article 10 may comprise symmetrical channel(s) in relation to the transverse center line and/or the longitudinal center line LCL

In these embodiments where the channel consists of one central section, four lateral sections, four diagonal sections and one or two closing section(s), the central section is preferably longitudinally centered on a point 414 which is at a distance from the front transverse edge 3 along the longitudinal center line LCL of between 30% and 55%, more preferably between 35% and 53%, even more preferably between 40% and 50%, of the absorbent core length (L). This advantageously allows the miction point positioning to correspond to the central section, so that liquid distribution towards front and back may quickly start.

In these same embodiments, preferably, the central section 41 has a length 413 of between 2 and 25% or between 4 and 25%, more preferably between 5 and 20%, even more preferably between 6 and 15 %, most preferably between 7 and 12%, of the absorbent core length L. Preferably, the ratio between the central section length 413 to the absorbent core length L is greater for the absorbent core 1 of the pant-type absorbent article 10 than for the absorbent core 1' of the pad-type absorbent article 10. We have indeed found that if the central section is too short, the absorption time may increase and rewet, to some extent, also may increase. When the central section is too long, it has been found that both absorption time and rewet may greatly increase. Advantageously the length of the channel CL taken along the longitudinal center line LCL is between 50 and 90%, preferably between 55 and 80%, more preferably between 60 and 75%, of the absorbent core length L. Preferably, the ratio between the channel length taken along the longitudinal center line LCL to the absorbent core length L for the absorbent core 1 of the pant-type absorbent article 10 is substantially equal to said ratio for the absorbent core 1' of the pad-type absorbent article 10'.

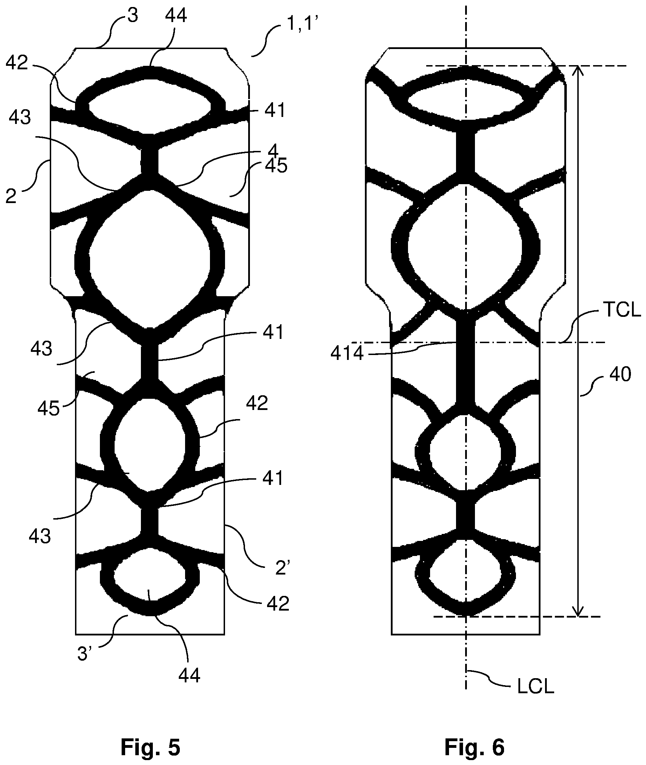

In alternative embodiments, as illustrated in Fig. 5 or 6, the channel 4 comprises at least two central sections 41, six lateral sections 42, eight diagonal sections 43 and two closing sections 44. Such pattern may provide a powerful draining system and/or comfort to the wearer. We have indeed found that all the channel sections may work together to offer powerful liquid distribution and may act as folding lines for the core, and the article including such core, to fit to the wearer body.

In these alternative embodiments where the channel comprises at least two central sections, the central section 41 closest to or crossing the transverse center line TCL, preferably the central section closest to or including the miction point, is preferably longitudinally centered on a point 414 which is at a distance from the front transverse edge along the longitudinal center line LCL of between 40% and 65%, more preferably between 45% and 60%, of the absorbent core length L. This advantageously allows the miction point positioning to correspond to the central section, so that liquid distribution towards front and back may quickly start.

In these alternative embodiments, preferably, the central section 41 closest to or crossing the transverse center line TCL, preferably the central section closest to or including the miction point, has a length 413 of between 5 and 25%, more preferably between 10% and 20%, of the absorbent core length L. We have indeed found that if the central section is too short, the absorption time may increase and rewet, to some extent, also may increase. When the central section is too long, it has been found that both absorption time and rewet may greatly increase.

In these alternative embodiments, the channel 4 may advantageously further comprise at least two diverging sections 45 connecting lateral sections 42 and/or diagonal sections 43 to the longitudinal edges 2,2' of the absorbent core 1,1'. These diverging sections may further help the liquid distribution through the entire core and/or improve the comfort and fit. Preferably, however, the absorbent core is free of any diverging section connecting the channel to the front or back transverse edges to reduce the risk of leakage in these zones.

In these alternative embodiments, the length of the channel CL taken along the longitudinal center line LCL is preferably between 70 and 95%, more preferably between 80 and 95%, even more preferably between 83 and 93% of the absorbent core length L.

In these alternative embodiments, the ratio of the channel surface area, i.e. the surface area substantially free of absorbent material in black in Fig. 3 or 4, to the surface area of absorbent material in white in Fig. 3 or 4 is preferably between 20 and 50%, more preferably between 30 and 40%. It has indeed been found that when this ratio is too low there might be not enough or not efficient enough fluid transport, and if the ratio is too high there might be a greater likelihood of leakage as liquid will flow faster to the edges of the core compared to the speed of acquisition into the absorbent material.

In accordance with the invention, the channel sections may be straight lines or may be curved lines. Preferably, the closing section is substantially curvilinear in shape, for example in the form of a U-bend with its convex side facing the absorbent core transverse edge at closest proximity, or is substantially linear in shape, for example forming a straight or triangular shape between the lateral sections end points.

According to the invention, preferably, the absorbent core is free of any channel or channel section extending up to the front and back transverse edges of the absorbent core. This has been found advantageous in that it creates transversally extending zones of absorbent material acting as barriers to absorb liquid before it reaches the front and back end of the core, thereby avoiding leakage in those generally risky areas of the core. A similar advantage may be reached, alternatively or additionally, by ensuring that the concentration of superabsorbent polymers within the core is higher closer to the front and back transverse edges compared to the central region closer to the transverse center line, for example by ensuring an increasing gradient of superabsorbent polymers concentration from the central portion of the core to the front and back portions. Alternatively or additionally, it might be desirable to have a concentration of superabsorbent polymers within the core higher around the miction point. This may offer a reservoir for absorption in order to minimize risk of leakage.

Advantageously, the lateral sections 42 of the first longitudinal portion may be aligned on a first longitudinal axis parallel to the longitudinal center line LCL, and the lateral sections 42 of the second longitudinal portion may be aligned on a second longitudinal axis parallel to the longitudinal center line LCL. This may render the manufacturing process easier, ensuring the adhesion of the core wrap top and bottom layers along lines parallel to the manufacturing direction. Preferably the channel is symmetric about the longitudinal center line.

According to the invention, the one or more channels are formed by the adhesion of the top layer of the core wrap with the bottom layer of said core wrap, in zones substantially free of absorbent material. Such adhesion may advantageously provide core stability and for example reduce the risk of sagging. This adhesion may be provided by any means known in the art to bond core wrap layers together, for example with adhesive or mechanical bonding.

In an embodiment, the top and bottom core wrap layers are adhered by one or more adhesives, *i.e.* adhesive bonding. The adhesive may be either uniformly applied within the channels or it may be applied in zones of the channels such to form zones, preferably alternating zones, of different bonding strength between the core wrap layers. Ways to achieve stronger bonding strength in some zones may include using higher amounts of adhesive in said zones, applying greater mechanical pressure on said zones, or utilizing a different adhesive type.

In an embodiment, the top and bottom core wrap layers are adhered along the channels, at a plurality of discrete joining areas, preferably wherein said discrete joining areas are free of adhesive and typically comprise mechanical bonds. Advantageously, this allows for a permanent bonding of the top and bottom layers of the core wrap that limits the presence of additional hydrophobic substances such as adhesives therebetween whilst maximizing the unbonded spaces therebetween to enhance the fluid flow through the channel.

In an embodiment the bonding strength in some zones of the channels is less than in others, and the top core wrap layer and bottom core wrap layer may separate in said zones. This arrangement may allow the bonding in some zones to fail upon for example swelling of the SAP such to allow more volume to be available for expansion thereof (and prevent early saturation or non-optimal absorption), with typically the zones resisting such expansion providing integrity of the channels even in wet state.

The core wrap layers are preferably nonwoven webs. Advantageously, at least one of the top and bottom core wrap layers is an elastic nonwoven (e.g. containing an elastic material such as Vistamaxx resin from ExxonMobil, or other suitable polymers capable of imparting elasticity to a nonwoven web). An advantage of this embodiment is that the nonwoven web better and more easily wraps around the 3D insert during the manufacturing process.

According to the invention, the absorbent core comprises absorbent material selected from the group consisting of cellulose fibers, superabsorbent polymers and combinations thereof. The absorbent material may in particular comprise cellulosic fluff and/or fibrous web typically comprising airlaid fibers of the cellulosic kind. The superabsorbent polymers may typically be in the form of a plurality of discrete particles that may be distributed within the absorbent material or directly agglomerated in one or more pockets between at least two nonwoven webs. The absorbent core may include cellulose fibers and superabsorbent polymers in a proportion of 5-55 Wt% cellulose and 45-95 Wt% SAP.

In an embodiment, in addition to the channel having the sections as described above, the core further comprises one or more disconnected channels, an advantage being effective added local uniform fluid distribution.

In an embodiment, the width of the channels is constant throughout a continuous path of a channel. Alternatively, it may vary along a channel. The width of the channels may be between 3 and 20 mm, preferably between 5 and 15 mm, more preferably between 5 and 12 mm.

The cores herein may have various shapes. Preferably the width of the core in the region of the transverse center line is less than in at least one of the front or back portions. This region may be positioned in a crotch portion of the absorbent article such to provide better ergonomics and fit along the leg of a wearer. It is preferred that said cores are symmetric at least about the longitudinal axis thereof. Irrespective of the core geometry, it is understood herein that the same or similar channels as described herein may be interchangeably used.

In an embodiment, the absorbent core is a multi-layer core comprising at least a first and a second distinct core layers positioned one on top of the other. The first and/or the second core layer may comprise at least one channel according to the invention. Preferably they both comprise at least one channel according to the invention, wherein the shape and/or dimensions and/or positioning of said channel is substantially identical in both core layers.

Advantageously, a first core layer comprises a first concentration of superabsorbent polymers therein and a second core layer comprises a second concentration of superabsorbent polymers therein. Said first and second concentrations may be different. The multi-layered core arrangement with differential superabsorbent polymer concentration enables to absorb fluids more efficiently in a multi-fold way and reduce the risk of gel-blocking. The second concentration of superabsorbent polymers, in the bottom core layer, may be greater than the first concentration of superabsorbent polymers, in the top core layer. Preferably said second concentration is at least 1.5, preferably 2, times greater than the first concentration. Such brings advantages such as reduced risk of gel-blocking. The type of superabsorbent polymers may be selected to ensure that the top core layer includes SAP performing well under pressure, to provide a better rewet, and that the bottom core layer includes SAP having fast absorption characteristics. The top core layer may include cellulose fibers and superabsorbent polymers in a proportion of 20-40 Wt% cellulose and 60-80 Wt% SAP; the bottom core layer may include cellulose fibers and superabsorbent polymers in a proportion of 15-30 Wt% cellulose and 70-85 Wt% SAP.

Preferably, the amount of SAP in the pad-type absorbent article 10' is lower than the amount of SAP in the pant-type absorbent article 10.

Absorbent articles according to the present invention include disposable incontinence diapers or diaper pants and incontinence pads. They comprise an absorbent core sandwiched between a liquid permeable topsheet and a liquid impermeable backsheet. The backsheet may comprise a print or graphic viewable from the garment facing side of said article that substantially matches the shape and/or contour of the channel(s). This has the advantage to further accentuate the visual perception of the presence of such channel and its location in the absorbent article.

The absorbent articles may further comprise an acquisition distribution layer (ADL), also referred to herein as acquisition and distribution system, positioned between said topsheet and said core. The ADL may be positioned at a body-facing side of the absorbent core, between the topsheet and the absorbent core of the absorbent article, and more preferably in close proximity or even in good contact (most preferably in direct contact) with the body-facing side of the absorbent core. The use of an ADL in combination with the channels of the present invention may lead to an extremely good distribution of fluids from a discharge area to the entire absorbent core whilst attaining excellent perceived dryness performance.

The acquisition distribution system may be a single layer of spunbond and/or carded (e.g. carded thermobonded) nonwoven. Alternatively, the acquisition distribution system may be multi-layered and comprise at least one spunbond layer and at least one meltblown layer typically the layers being nonwoven layers. Preferably, the acquisition distribution system is a nonwoven selected from the group consisting of: SM, SMS, SMMS, and combinations thereof.

In an embodiment, the acquisition distribution layer comprises a plurality of layers, wherein at least one of said layers, preferably each of said layers, consists of spunbond, meltblown and/or carded (e.g. thermocarded) nonwoven and wherein at least the layer most distal from the body-facing side of the absorbent core consists of spunbond and/or carded nonwoven, preferably wherein both the layer most distal and the layer most proximal to the body-facing (also referred to herein as "skin-facing") side of the absorbent core consists of spunbond and/or carded nonwoven.

In an embodiment, the acquisition distribution layer for use herein comprises synthetic fibers which are comprised at a level of greater than 80%wt by weight of said acquisition distribution layer. The acquisition distribution layer may have a basis weight of from 5 to 50 g/m², 10 to 50 g/m², preferably from 15 to 40 g/m², more preferably from 18 to 35 g/m², even more preferably from 20 to 30 g/m², most preferably from 21 to 25 g/m².

In an embodiment, the acquisition distribution system is the top layer of the core wrap and the absorbent article is free of additional layers, such as an acquisition distribution layer, so that the top layer of the core wrap is in direct contact with the topsheet. Said top layer of the core wrap may comprise a spunbond and/or carded, e.g. carded thermobonded, nonwoven layer comprising synthetic fibers, wherein preferably said synthetic fibers are comprised at a level of greater than 80%wt by weight of said layer, and/or wherein said top layer of the core wrap has a basis weight of from 5 to 50 g/m². Alternatively, said top layer of the core wrap may be multi-layered and comprise at least one spunbond layer and at least one meltblown layer typically the layers being nonwoven layers. Preferably, the multi-layered top layer of the core wrap acting as acquisition distribution system is a nonwoven selected from the group consisting of: SM, SMS, SMMS, and combinations thereof. Advantageously, by choosing core wraps as described, improved performance on rewet may be achieved even whilst eliminating the presence of further acquisition distribution layers, thus further introducing cost benefits.

We have found that fluid distribution, in embodiments of the present absorbent articles which comprise an ADL, may depend on the relative size and positioning of the ADL with respect to the fluid distribution structure, and in particular the channel(s), of the absorbent core. The acquisition distribution layer may be asymmetrically positioned over the absorbent core, offset at least along the longitudinal axis, preferably positioned such that it does not overlap a portion of the channel at a position proximal to the back of the core, more preferably wherein the acquisition distribution layer is positioned such that it does not overlap with a closing section of the channel. This arrangement has the advantage of raw material cost saving by ensuring the ADL is positioned where it is needed most. Moreover by ensuring a part of the channel remains exposed (particularly the closing section of the channel) speed of liquid flow through the channel from front to back is not compromised.

## Claims

1. Array of disposable absorbent articles adapted for different activities, the array comprising:
a. a first package comprising a plurality of pant-type absorbent articles (10), configured to be worn about the lower torso of a wearer, said pant-type absorbent articles (10) comprising
i. a chassis (2) comprising a front waist region (11), a back waist region (12) each of said regions being provided with longitudinal edges (13, 14, 15, 16) and a transversal edge (17, 18), and a crotch region (19) extending between said front and back waist regions (11,12);
ii. a liquid-absorbent member (20) extending across the crotch region (19) in the direction of a longitudinal center line (LCL) of the pant-type absorbent article (10) into the front and back waist regions (11,12), the liquid-absorbent member comprising a liquid permeable topsheet arranged on a body-facing side, a liquid impermeable backsheet arranged on a garment-facing side and an absorbent core (1) arranged in between said topsheet and backsheet; and
iii. wherein the crotch region (19), the front waist region (11) and the back waist region (12) define leg openings (22) of the absorbent article (10), the front waist region (11) and the back waist region (12) having an edge contour (23, 24) extending starting from the longitudinal edges (13, 14, 15, 16) towards a transverse center line (TCL) of the crotch region (19);
iv. wherein said front waist region (11) and back waist region (12) comprise first elastification means (25), and second elastification means (22, 23) are provided in an area (26,27) of the front waist region (11) and the back waist region (12) on the crotch side facing the leg openings (19), said second elastification means (22, 23) extend starting from longitudinal edges (13, 14, 15, 16) towards the longitudinal center line (LCL) of the absorbent article (10); and
b. a second package comprising a plurality of pad-type absorbent articles (10'), configured to be fastened to an undergarment, said pad-type absorbent article (10') comprising:
i. a liquid permeable topsheet (31) arranged on a body-facing side;
ii. a liquid impermeable backsheet (32) arranged on a garment-facing side comprising fastening means to secure the pad-type absorbent article (10') to an undergarment; and
iii. an absorbent core (1') arranged in-between said topsheet (31) and backsheet (32),
c. wherein the absorbent core (1,1') of said pant-type and said pad-type absorbent articles (10,10') comprises absorbent material selected from the group consisting of cellulose fibers, superabsorbent polymers and combinations thereof, said absorbent material being contained within at least one core wrap substrate enclosing said absorbent material, and wherein a top layer of said core wrap is adhered to a bottom layer of said core wrap to form one or more channels (4) substantially free of said absorbent material.

2. Array of disposable absorbent articles (10,10') according to claim 1, wherein the one or more channels (4) of the absorbent core (1) of the pant-type absorbent articles (10) have the same shape as the one or more channels (4) of the absorbent core (1') of the pad-type absorbent articles (10').

3. Array of disposable absorbent articles (10,10') according to claim 2, wherein the pant-type absorbent articles (10) and the pad-type absorbent articles (10') each comprise symmetrical channel(s) (4).

4. Array of disposable absorbent articles (10,10') according to claim 1, wherein the one or more channels (4) of the absorbent core (1) of the pant-type (10) absorbent articles have a different shape than the one or more channels (4) of the absorbent core (1') of the pad-type absorbent articles (10').

5. Array of disposable absorbent articles (10,10') according to claim 4, wherein each pant-type absorbent article (10) comprises asymmetrical channel(s) (4) and each pad-type absorbent article (10') comprises symmetrical channel(s) (4).

6. Array of disposable absorbent articles (10,10') according to any of the preceding claims, wherein the pant-type absorbent articles (10) comprise a first channel area to core area ratio and the pad-type absorbent articles (10') comprise a second channel area to core area ratio, wherein the first channel area to core area ratio is lesser than the second channel area to core area ratio, preferably at least 15% lesser, preferably at least 20% lesser, more preferably at least 30% lesser, more preferably between 40% and 70% lesser.

7. Array of disposable absorbent articles (10,10') according to any of the claims 1 to 6, wherein said absorbent core (1,1') of said pant-type and/or said pad-type absorbent article (10,10') having a first and second longitudinal edge (2,2'), a front and back transverse edge (3,3'), and a longitudinal center line (LCL) dividing the absorbent core (1,1') in a first longitudinal portion and a second longitudinal portion on either side of the longitudinal center line (LCL), said absorbent core (1,1') having a transverse center line (TCL) dividing the absorbent core (1,1') in a front portion and a back portion on either side of the transverse center line (TCL), thereby forming four quadrants, wherein at least one channel (4) follows a substantially continuous path from any point of said channel to any other point of the same channel, **characterized in that** said channel (4) comprises at least:
- one longitudinally extending central section (41) coincident with the longitudinal center line (LCL),
- one longitudinally extending lateral section (42) in each of the four quadrants,
- four diagonally extending diagonal sections (43), diverging from the central section end points (411,412) towards the lateral sections in each quadrant and connecting said central (41) and lateral (42) sections, and
- one transversally extending closing section (44,44'), in one of the front or back portions, joining each of the two lateral sections end points (421,422) closest to the absorbent core respective front or back transverse edge (3,3').

8. Array of disposable absorbent articles (10,10') according to claim 7, wherein the absorbent core (1,1') of said pant-type and/or said pad-type absorbent article (10,10') comprises a channel (4) comprising a second transversally extending closing section (44,44'), in the other of the front or back portions, joining each of the two lateral sections end points (421,422) closest to the absorbent core respective front or back transverse edge (3,3').

9. Array of disposable absorbent articles (10,10') according to claim 7 or 8, wherein said channel (4) consists of one central section (41), four lateral sections (42), four diagonal sections (43) and one or two closing section(s) (44).

10. Array of disposable absorbent articles (10,10') according to claim 9, wherein the central section (41) is longitudinally centered on a point (414) which is at a distance from the front transverse edge (3) along the longitudinal center line (LCL) of between 30% and 55% of the absorbent core length (L).

11. Array of disposable absorbent articles (10,10') according to any of the claims 9 to 10, wherein the central section (41) has a length (413) of between 4 and 25% of the absorbent core length (L), wherein the ratio between the central section length (413) to the absorbent core length (L) is greater for the absorbent core (1) of the pant-type absorbent article (10) than for the absorbent core (1') of the pad-type absorbent article (10').

12. Array of disposable absorbent articles (10,10') according to any of the claims 9 to 11, wherein the length of the channel (CL) taken along the longitudinal center line (LCL) is between 50 and 90% of the absorbent core length (L) wherein the ratio between the channel length taken along the longitudinal center line (LCL) to the absorbent core length (L) for the absorbent core (1) of the pant-type absorbent article (10) is substantially equal to said ratio for the absorbent core (1') of the pad-type absorbent article (10').

13. Array of disposable absorbent articles (10,10') according to any of the preceding claims, wherein the lateral sections (42) of the first longitudinal portion are aligned on a first longitudinal axis parallel to the longitudinal center line (LCL), and the lateral sections (42) of the second longitudinal portion are aligned on a second longitudinal axis parallel to the longitudinal center line (LCL).

14. Array of disposable absorbent articles (10,10') according to any of the preceding claims, wherein the liquid permeable topsheet is treated with a hydrophilic agent, preferably comprising anionic and/or zwitterionic agents.

15. Array of disposable absorbent articles (10,10') according to claim 14, wherein only part of the topsheet is treated and the treated zone is positioned over the channel (4), preferably the width of the treated zone corresponding to the maximum width (46) of the channel, as measured along a direction parallel to the transverse center line.
